# EUROPEAN PATENT APPLICATION

(11) **EP 2 368 593 A1**
(43) Date of publication of application: **28.09.2011**
(21) Application number: 10158040.5
(22) Date of filing: 26.03.2010
(51) Int. Cl.: A61M 16/00

(54) **Estimating a leakage flow**

(71) Applicant: Dräger Medical GmbH, 23558 Lübeck (DE)
(72) Inventor: Robben, Koen, 5046, RK Tilburg (NL)
(74) Representative: UEXKÜLL & STOLBERG

(57) **Abstract**

The invention relates to a medical device 100 for estimating a leakage flow 160 that occurs during ventilation of a patient 120, the medical device comprising a first estimation unit 101 arranged for estimating the leakage flow in dependence on a measured airway pressure and a leakage parameter, a second estimation unit 102 arranged for estimating a patient airway flow 170 in dependence on the leakage flow and a measured ventilation flow 150, an adjustment unit 103 arranged for adjusting the leakage parameter in dependence on the patient airway flow to provide an adjusted leakage parameter, and a third estimation unit 104 arranged for re-estimating the leakage flow in dependence on the adjusted leakage parameter.

## Description

### FIELD OF THE INVENTION

The invention relates to a method and a medical device for estimating a leakage flow that occurs during ventilation of a patient. The invention further relates to a computer program product comprising instructions for causing a processor system to perform said method.

### BACKGROUND OF THE INVENTION

Ventilators are used in modern medicine to assist or replace spontaneous breathing of a patient. For that purpose, the ventilator may be connected to the patient by means of a patient circuit and be arranged for supplying breathing gas to the patient through the patient circuit. The patient circuit typically comprises a conduit for transporting the breathing gas, and is connected to the patient through, for example, an endotracheal tube or a breathing mask.

During ventilation of the patient, the breathing gas may leak from the patient circuit before reaching the patient's airways. For example, a breathing mask may be used to perform non-invasive ventilation of a patient. Breathing masks seldom fit the patient's face perfectly, and thus may lead to leakage of breathing gas. Furthermore, the mask may shift during ventilation, which changes the characteristics of the leakage. As a result, the amount of breathing gas reaching the patient's airways cannot be controlled accurately.

It is known that the accuracy of controlling the amount of breathing gas supplied to the patient can be improved by estimating the leakage of breathing gas, and subsequently compensating for the estimated leakage during ventilation of the patient.

US 2007/0144522 describes a method for determining leaks of a respirator. The method comprises determining a leak volume *V_{Li}* using a leakage model that comprises a product of a recorded inspiration pressure *pᵢₙₛₚ* and an inspiration time *Iᵢₙₛₚ* in accordance the equation *V_{Li}=A·pᵢₙₛₚ^{B}·Iᵢₙₛₚ.* In this leakage model, the parameters A and B are determined using a regression model with the recorded inspiration pressure *pᵢₙₛₚ* as the regressor.

### SUMMARY OF THE INVENTION

A problem of US 2007/0144522 is that, in order to estimate the leak volume, the inspiratory pressure has to be varied during consecutive breath cycles of the patient. The inspiratory pressure during ventilation is ideally determined solely by medical factors. The necessity of having to vary the inspiratory pressure for purpose of the regression model thus leads to a compromise between the demands imposed by medical factors, and the demands imposed by using the inspiratory pressure as regressor in a regression model.

It would be advantageous to have an improved method for estimating the leakage occurring during ventilation of a patient.

To better address this concern, a first aspect of the invention provides a method of estimating a leakage flow that occurs during ventilation of a patient, the method comprising estimating the leakage flow in dependence on a measured airway pressure and a leakage parameter, the leakage parameter being indicative of a relation between the measured airway pressure and the leakage flow, estimating a patient airway flow in dependence on the leakage flow and a measured ventilation flow, adjusting the leakage parameter in dependence on the patient airway flow to provide an adjusted leakage parameter, and re-estimating the leakage flow in dependence on the adjusted leakage parameter.

In a related aspect of the invention, a medical device for estimating a leakage flow that occurs during ventilation of a patient comprises a first estimation unit arranged for estimating the leakage flow in dependence on a measured airway pressure and a leakage parameter, the leakage parameter being indicative of a relation between the measured airway pressure and the leakage flow, a second estimation unit arranged for estimating a patient airway flow in dependence on the leakage flow and a measured ventilation flow, an adjustment unit arranged for adjusting the leakage parameter in dependence on the patient airway flow to provide an adjusted leakage parameter, and a re-estimation unit arranged for re-estimating the leakage flow in dependence on the adjusted leakage parameter.

The abovementioned aspects of the invention allow the leakage flow to be estimated as a function of at least a measured airway pressure and a leakage parameter. The measured airway pressure may be obtained from a pressure sensor near the patient's airways. The leakage parameter determines how the measured airway pressure relates to the leakage flow, and thus allows the leakage flow to be estimated.

The leakage flow expresses how much of a ventilation flow provided by a medical device leaks away before reaching the patient's airways. The ventilation flow may be measured by the medical device that provides said flow. Hence, given a measured ventilation flow and an estimate of the leakage flow, the patient airway flow may be estimate by compensating in the measured ventilation flow for the leakage flow that occurs.

The method and the medical device make use of the insight that the estimate of the patient airway flow is indicative of the accuracy of the estimate of the leakage model, and thus whether the leakage model accurately models the actual leakage flow. The reason for this is that there exists an expectancy or norm of the patient airway flow. If the estimate of the patient airway flow has a characteristic that deviates significantly from the norm, this is likely caused by an inaccurate leakage model.

If the leakage model is found to be inaccurate, this is likely the result of an inaccurate leakage parameter since the airway pressure is typically measured with a relatively high reliability. The leakage parameter is thus adjusted in dependence on the patient airway flow to obtain an adjusted leakage parameter. The leakage flow is then re-estimated using said adjusted parameter to obtain an improved estimate of the leakage flow.

Consequently, the estimate of the patient airway flow is used to improve the estimate of the leakage flow. It is therefore not needed to vary the inspiratory pressure to determine an accurate leakage model, and thus an accurate estimate of the leakage flow. The inspiratory pressure can therefore be solely determined by medical factors. Furthermore, a processor system performing said method may suffice with a relatively low computational performance, whereas in contrast, solving a regression model typically needs a processor system with a relatively high computational performance.

In an embodiment, the adjusting the leakage parameter comprises determining a summary value in dependence on the patient airway flow during a portion of a breath cycle of the patient, comparing the summary value with a reference value to obtain a comparison result, and adjusting the leakage parameter in dependence on the comparison result.

The patient airway flow may be most indicative of the accuracy of the leakage flow during a portion of a breath cycle of the patient. By determining a summary value in dependence on the patient airway flow during that portion, the accuracy of the leakage flow is indicated by a single value. The summary value is then compared against a reference value, with the reference value being indicative of a norm of the summary value. As such, the result of the comparison indicates a deviation of the summary value from the norm. The leakage parameter is then adjusted in dependence on the result of the comparison in order to improve the leakage model. Consequently, the deviation of the summary value from the norm is used to improve the accuracy of the leakage model and thus the estimate of the leakage flow.

In an embodiment, the determining the summary value comprises averaging the patient airway flow during the portion of the breath cycle of the patient.

An average value may express relevant characteristics of the patient airway flow by means of just a single value. Furthermore, an average of the patient airway flow limits the influence of errors in the estimate of the patient airway flow, since single erroneous values only have a limited contribution in an average of multiple values.

In an embodiment, the estimating the leakage flow comprises estimating the leakage flow using a leakage model in which the leakage parameter is used in an exponent of the measured airway pressure.

The leakage flow may be modeled by a leakage model. Furthermore, the relation between measured airway pressure and leakage flow is seldom linear. With the leakage parameter being used in an exponent of the measured airway pressure, non-linear relations can be taken into account in the leakage model.

In an embodiment, the leakage model comprises the equation *flowₗₑₐₖ=A·p_{aw}^{B}* in which *flowₗₑₐₖ* is the leakage flow, *A* a scaling factor, *p_{aw}* the measured airway pressure and *B* the leakage parameter.

The estimate of the leakage flow may be improved by a leakage model that comprises a scaling factor expressing the linear relation between the measured airway pressure and the leakage flow, as well as a leakage parameter expressing the non-linear relation. This model allows a more accurate representation of the actual leakage flow than a leakage model comprising either the scaling factor or the leakage parameter.

In an embodiment, the estimating the patient airway flow comprises subtracting the leakage flow from the measured ventilation flow.

The measured ventilation flow is typically the sum of the leakage flow and the patient airway flow. Hence, the patient airway flow is estimated by subtracting from the measured ventilation flow the estimate of the leakage flow.

In an embodiment, the breath cycle of the patient comprises an inspiratory phase and an expiratory phase, and the determining the summary value comprises determining the summary value in dependence on the patient airway flow during the portion of the inspiratory phase during which the patient airway flow is estimated to be expiratory, or the portion of the expiratory phase during which the patient airway flow is estimated to be inspiratory.

The patient airway flow has a different indicative value during the inspiratory phase than during the expiratory phase. Hence, the accuracy of the leakage model may be improved by determining the summary value from the patient airway flow specifically during the inspiratory phase or the expiratory phase.

Furthermore, it is of particular interest if the patient airway flow has a flow direction that is contrary to the flow direction suggested by the phase of the breath cycle, such as for example if the patient airway flow is estimated to be strongly inspiratory during the expiratory phase of the breath cycle. In this case, the leakage model and thus the estimate of the leakage flow is most likely inaccurate.

Hence, by determining the summary value in dependence on expiratory patient airway flow during the inspiratory phase or inspiratory patient airway flow during the expiratory phase during, the summary value has a relatively high indicative value. Consequently, the leakage parameter may be adjusted in dependence on the summary value to further improve the accuracy of the leakage model.

In an embodiment, the portion of the inspiratory phase or the expiratory phase comprises a point in a time interval from 75% to 95% of said phase.

The indicative value of the patient airway flow is relatively high in the time interval from 75% to 95% of the inspiratory phase or the expiratory phase. The reason for this is that the time interval is near the end of either phase, and thus the patient airway flow is typically relatively small. Consequently, any significant deviations from this norm may indicate an inaccurate leakage model.

In an embodiment, the determining the summary value comprises averaging the patient airway flow during the portion of the expiratory phase during which the patient airway flow is estimated to be inspiratory, the portion comprising the point in the time interval from 75% to 95% of the expiratory phase, the comparing the summary value comprising comparing the summary value with a lower reference value and with an upper reference value, and the adjusting the leakage parameter comprising decreasing the leakage parameter if the summary value is above the upper reference value, and increasing the leakage parameter if the summary value is below the lower reference value.

The patient airway flow during the expiratory phase of the breath cycle should be relatively small, as the patient will have exhaled most of the breathing gas that was breathed in during the inspiratory phase. Thus, if the patient airway flow is, for example, strongly inspiratory during the portion of the breath cycle, the leakage model is most likely inaccurate.

Moreover, there exists a relatively clear relationship between said patient airway flow and the leakage parameter, in that the leakage parameter is too high if the patient airway flow is above an upper threshold, and in that the leakage parameter is too low if the patient airway flow is below a lower threshold. The leakage parameter is therefore decreased in the former case and increased in the latter case. Consequently, the accuracy of the leakage model may be improved and thus the estimate of the leakage flow.

In an embodiment, the estimating the leakage flow comprises estimating the leakage flow in dependence on the measured airway pressure and the adjusted leakage parameter provided by the re-estimating the leakage flow from a previous execution of said method.

Performing the method during or after a breath cycle results in an adjusted leakage parameter. If the method is performed again in a subsequent breath cycle, the adjusted leakage parameter may be used as leakage parameter in the estimation of the leakage flow in the subsequent breath cycle. The reason for this is that the leak typically changes slowly in characteristics. Hence, the adjusted leakage parameter of a previous breath cycle is a relatively good prediction for the leakage parameter in the subsequent breath cycle.

In an embodiment, a computer program product comprises instructions for causing a processor system to perform the method of any one of the above embodiments.

The method steps according to the above embodiments are suitable for being performed by a processor system. For that purpose, the method steps may be implemented as a computer program product that comprises suitable instructions for the processor system.

In an embodiment, the medical device is a breathing device for non-invasive ventilation of a patient by means of at least one of the group of: a breathing mask, a tracheastoma, a mouth piece or a non-invasive ventilation helmet.

The leakage flow during non-invasive ventilation of a patient by means of one of the abovementioned devices is typically more severe than the leakage flow during invasive ventilation by means of, for example, an endotracheal tube. Hence, the control over breathing gas entering the patient airways is less accurate during non-invasive ventilation. This makes an accurate prediction of the leakage flow particularly relevant.

In an embodiment, the medical device further comprises a display for providing a status indication in dependence on the leakage flow.

A display enables the medical device to provide a caretaker of the patient with a visual status indication of the leakage flow and of aspects relating to the leakage flow.

### BRIEF DESCRIPTION OF THE DRAWINGS

These and other aspects of the invention are apparent from and will be elucidated with reference to the embodiments described hereinafter. In the drawings,
- Fig. 1: shows a medical device ventilating a patient through a patient circuit;
- Fig. 2: shows a flowchart of a method for estimating a leakage flow;
- Fig. 3: shows a flowchart of a method for estimating the leakage flow;
- Fig. 4: shows a graph of a reference, estimated and re-estimated patient airway flow;
- Fig. 5: shows a graph of a reference, estimated and re-estimated leakage flow;
- Fig. 6: shows a graph of a measured ventilation flow during a breath cycle;
- Fig. 7: shows a graph of a patient airway flow during a breath cycle.

### DETAILED DESCRIPTION OF EMBODIMENTS

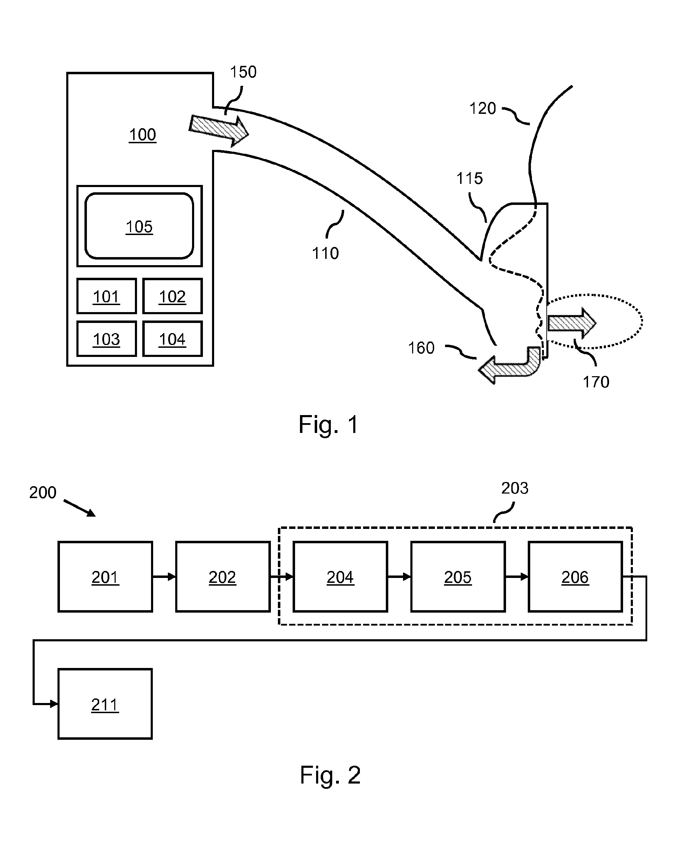

Fig. 1 shows a medical device 100 that is suitable for estimating a leakage flow 160 that occurs during ventilation of a patient 120. The medical device 100 is connected to the patient 120 through a patient circuit 110, and the patient circuit is arranged for transporting breathing gas between the medical device 100 and the patient 120. During ventilation of the patient 120, the medical device 100 provides a flow of breathing gas, with the amount of flow typically being measured by the medical device 100. Fig. 1 thus shows the medical device 100 providing a measured ventilation flow 150 to the patient circuit 110.

The patient circuit 110 is connected to the patient 120 during ventilation. For this purpose, the patient circuit 110 may comprise an endotracheal tube that enters the patient's trachea. The medical device and the patient circuit may also be arranged for non-invasive ventilation of the patient 120, as is shown in Fig. 1. For this purpose, the patient circuit 110 comprises a breathing mask 115 for covering the patient's mouth and nose. Alternatively, the patient circuit 110 may comprise a tracheastoma, a mouth piece, a non-invasive ventilation helmet or a similar device for non-invasive ventilation of the patient 120.

In case of a perfectly fitting breathing mask, the measured ventilation flow 150 results in an approximately equal amount of flow reaching the patient's airways. The breathing mask 115, however, typically does not fit the patient's face perfectly, thereby resulting in a leakage flow 160 occurring near the patient 120. This means that the patient airway flow 170 cannot be controlled accurately by the medical device.

To improve the accuracy of controlling the patient airway flow 170, the medical device 100 comprises a first estimation unit 101 for estimating the leakage flow, a second estimation unit 102 for estimating the patient airway flow, an adjustment unit 103 for adjusting the leakage parameter, and a re-estimation unit 104 for re-estimating the leakage flow. The functionality of each respective unit is similar to the method steps shown in Fig. 2, and will thus be covered in the description of Fig. 2 together with the method steps.

To provide the caretaker of the patient 120 with status information regarding the leakage flow 160, the medical device 100 further comprises a display 105. The display 105 may show the leakage flow 160 in various ways, for example by expressing the leakage flow in Liters/minute or as a percentage of the measured ventilation flow. The status information may also include other relevant aspects regarding the ventilation of the patient.

Fig. 2 shows a method 200 for estimating a leakage flow that occurs during ventilation of a patient. As a first step, the method 200 comprises estimating 201 the leakage flow in dependence on a measured airway pressure and a leakage parameter in an "Estimate the leakage flow" step. This step corresponds in terms of functionality to the first estimation unit 101 of the medical device 100 shown in Fig. 1.

The measured airway pressure is the pressure that is measured in or near the patient's airways. Said airway pressure may be measured by placing a pressure sensor near the patient's airways, for example near the patient's nose or mouth. The airway pressure may be measured with a relatively high reliability, as the leakage flow occurring nearby has only limited influence on the airway pressure.

The leakage parameter indicates how the measured airway pressure relates to the estimate of the leakage flow. This effect is frequently non-linear. Hence, the leakage parameter may be an exponent of the measured airway pressure to model this non-linear effect. The measured airway pressure may also affect the estimate of the leakage flow in a linear manner. Hence, the leakage parameter may be a scaling factor to model the linear effect. The leakage model may also comprise a scaling factor that models the linear effect next to a leakage parameter that models the non-linear effect.

The leakage model may take the form of the equation *flowₗₑₐₖ=Ap_{aw}^{B},* with *flowₗₑₐₖ* being the leakage flow, *A* the scaling factor, *p_{aw}* the measured airway pressure and *B* the leakage parameter. In this leakage model, the leakage parameter models the non-linear relation between measured airway pressure and the leakage flow, and the scaling factor models the linear relation. However, other leakage models that incorporate a linear and a non-linear relation between the measured airway pressure and the leakage flow may be equally suitable for the purpose of estimating the leakage flow.

The leakage flow may be estimated by entering the values of the scaling factor *A*, the measured airway pressure *p_{aw}* and the leakage parameter *B* into the leakage model, and subsequently calculating the estimate of the leakage flow *flowₗₑₐₖ.*

The method 200 further comprises estimating 202 a patient airway flow in an "Estimate the patient airway flow" step in dependence on the leakage flow and a measured ventilation flow. This step corresponds in terms of functionality to the second estimation unit 102 of the medical device 100 shown in Fig. 1.

In many ventilators, the flow going to the patient is measured bi-directionally with a flow sensor, thus providing a measured ventilation flow. Given the measured ventilation flow and an estimate of the leakage flow, the patient airway flow may be estimated as the patient airway flow together with the leakage flow is approximately equal to the measured ventilation flow. The step of estimating 202 the patient airway flow may thus comprise simply subtracting the leakage flow from the measured ventilation flow.

The above estimate of the patient airway flow may not be accurate in all cases. For example, the composition of the breathing gas is different before inspiration and after expiration of the patient. This results in the volume and thus the flow of breathing gas entering the patient's airways not being equal to the flow of breathing gas exiting said airways. Consequently, the estimate of the patient airway flow may be improved by taking this change in composition into account, for example by scaling the exhaled airflow with a composition correction factor. Similarly, the patient may have minor leaks in his lungs, which may also be taken into account in the estimate of the patient airway flow.

The method 200 further comprises adjusting 203 the leakage parameter in dependence on the patient airway flow in an "Adjusting the leakage parameter" step to provide an adjusted leakage parameter. This step corresponds in terms of functionality to the adjustment unit 103 of the medical device 100 shown in Fig. 1.

The patient airway flow is indicative of the accuracy of the leakage model, and thus of the estimate of the leakage flow. The leakage model may be adjusted by adjusting the leakage parameter. For example, an untypically large patient airway flow during expiration may be indicative that the leakage parameter should be decreased.

For this purpose, the step of the adjusting 203 the leakage parameter may comprise determining 204 a summary value in dependence on the patient airway flow during a portion of a breath cycle of the patient. The summary value may be determined in various ways, for example by averaging the patient airway flow during the portion of the breath cycle of the patient or by applying a digital filter to said patient airway flow.

The step of the adjusting 203 the leakage parameter may further comprise comparing 205 the summary value with a reference value to obtain a comparison result. The reference value is typically indicative of a norm of the summary value. For example, the summary value may be an average of the patient airway flow, and the reference value may be a threshold value of 50 ml/min (milliliters per minute). Hence, the average patient airway flow is compared to the threshold value of 50 ml/min to obtain a comparison result.

The step of the adjusting 203 the leakage parameter may lastly comprise adjusting 206 the leakage parameter in dependence on the comparison results. For example, if the average patient airway flow is above 50 ml/min, the leakage parameter may be decreased or may be set to a pre-determined value.

Lastly, the method 200 comprises re-estimating 211 the leakage flow using the adjusted leakage model in a "Re-estimating the leakage flow" step. This step corresponds in terms of functionality to the re-estimation unit 104 of the medical device 100 shown in Fig. 1, and typically comprises re-calculating the estimate of the leakage flow using the adjusted leakage parameter and the measured airway pressure that was used when previously estimating the leakage flow. In particular, this step may comprise the step 201 of "Estimating the leakage flow" of the method 200 being performed again in a subsequent breath cycle.

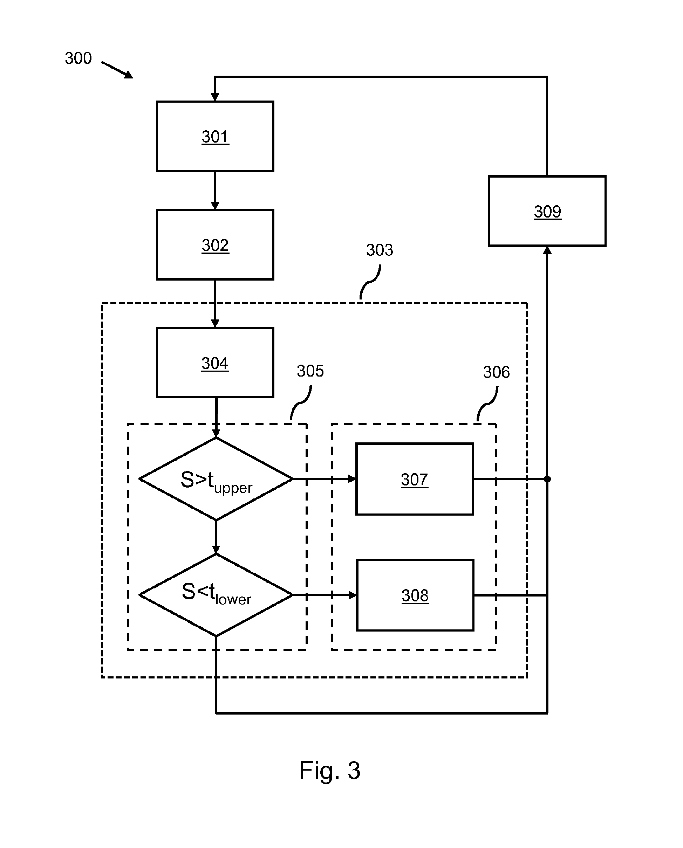

Fig. 3 shows a method 300 for estimating a leakage flow that occurs during ventilation of a patient. The method 300 comprises estimating 301 the leakage flow in dependence on a measured airway pressure and a leakage parameter in an "Estimate the leakage flow" step. This step may be identical to the step 201 "Estimating the leakage flow" as depicted in Fig. 2.

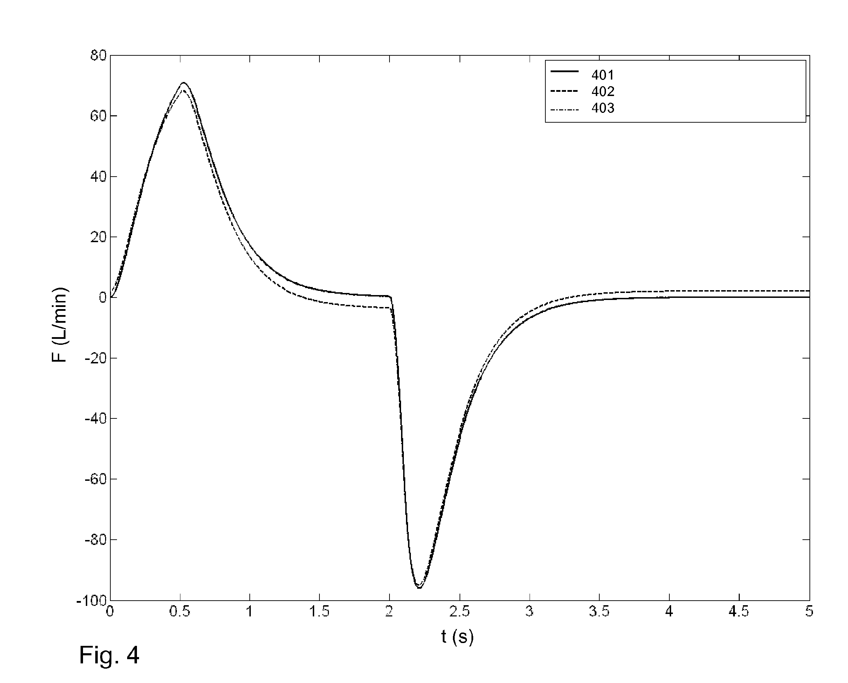

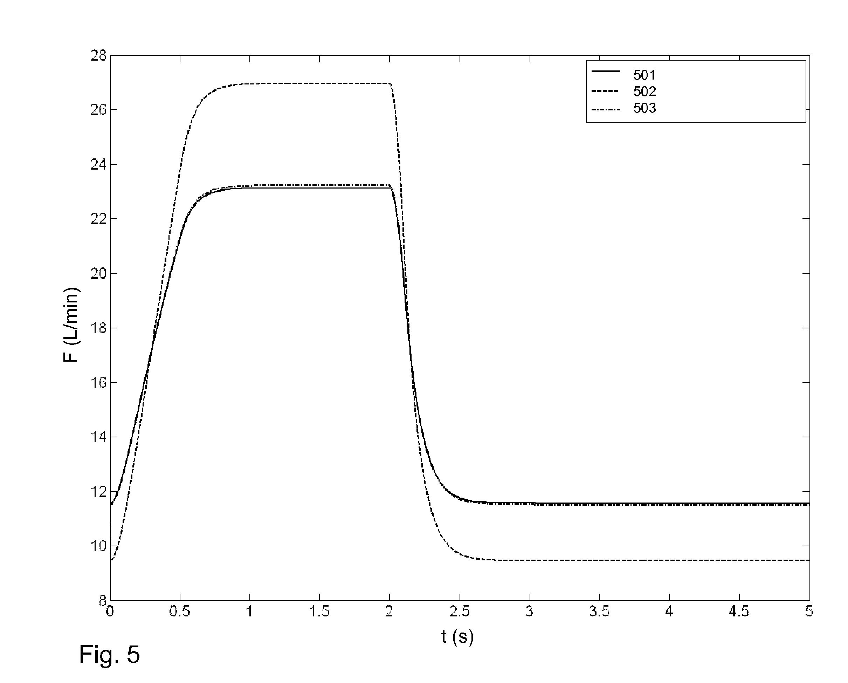

In addition, the leakage flow may be estimated for a given time instance or for a given time interval. An example of a leakage flow estimated for a full breath cycle is shown in Fig. 5 as a dashed line 502. In this Fig. and in Fig. 4, 6 and 7, the horizontal axis denotes the time during a breath cycle in seconds, and the vertical axis the leakage flow in Liters per minute. It can be seen that the leakage flow 502 is estimated to be large during the first, mostly inspiratory, half of the breath cycle, whereas the leakage flow 502 is estimated to be small during the second, mostly expiratory, half of the breath cycle.

The method 300 further comprises estimating 302 a patient airway flow in an "Estimate the patient airway flow" step in dependence on the leakage flow and a measured ventilation flow. This step may be identical to the step 202 "Estimate the patient airway flow" shown in Fig. 2. As such, the step of estimating 302 the patient airway flow may comprise simply subtracting the leakage flow from the measured ventilation flow.

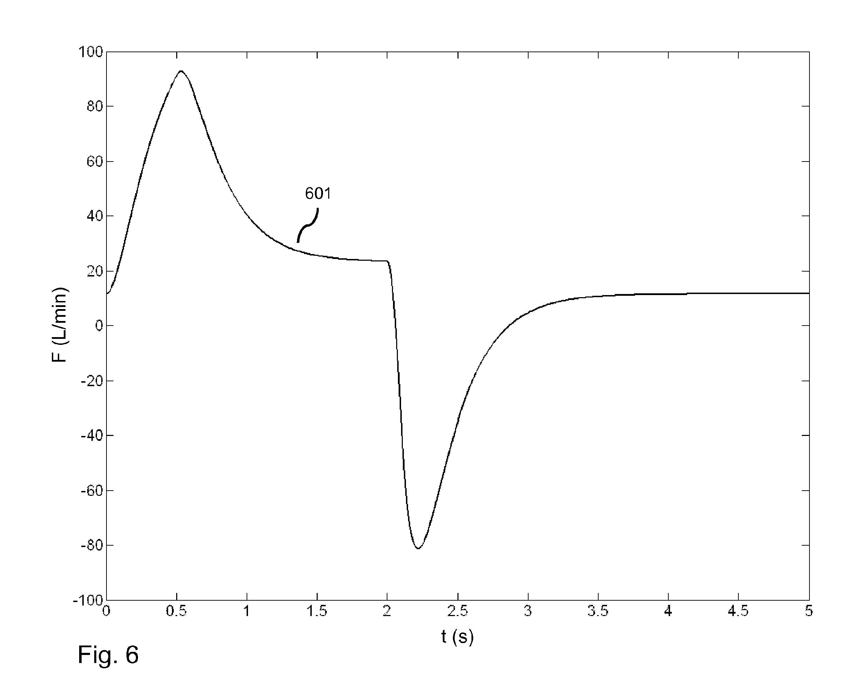

Similar to the leakage flow, the ventilation flow may be measured at a given time instance or during a given time interval. For example, the measured ventilation flow 601 may be provided for an entire breath cycle of the patient, as is shown in Fig. 6. As such, an estimate of the patient airway flow may be obtained by subtracting the leakage flow 502 shown in Fig. 5 from the measured ventilation flow 601 shown in Fig. 6. This results in the estimate of the patient airway flow 402 shown in Fig. 4 as a dashed line.

In this respect, it should be noted that positive patient airway flow corresponds to inspiration, i.e. the patient is breathing in, and negative patient airway flow corresponds to expiration, i.e. the patient is breathing out.

The patient airway flow may also be processed further. In particular, the exact value at a given time instance may be less relevant than the average of the patient airway flow during a given time-interval. An average allows the influence of outliers in the estimate of the patient airway flow to be reduced, since averaging multiple values limits the contribution of single erroneous values. Furthermore, an average may express relevant characteristics of the patient airway flow by means of just a single value.

The averaging may encompass a portion of the breath cycle that is of particular relevance. For example, the patient airway flow may be averaged during a portion of the breath cycle during which the patient airway flow is inspiratory or expiratory. Similarly, the patient airway flow may be averaged during a portion of the inspiratory phase or the expiratory phase of the breath cycle.

In this respect, it should also be noted that the portions during which the patient airway flow is inspiratory or expiratory do not necessarily coincide with the inspiratory and expiratory phases, respectively. The reason for this is that the leakage flow, and thus the patient airway flow, may be estimated erroneously. Hence, the estimate of the patient airway flow may comprise portions of inspiratory flow during the expiratory phase of the breath cycle, whereas typically the expiratory phase comprises mainly expiratory flow.

Various options exist for determining the inspiratory phase and the expiratory phase of the breath cycle. As a non-limiting example, the medical device providing the ventilation flow may be arranged for determining the time intervals corresponding to the inspiratory phase and the expiratory phase of the breath cycle.

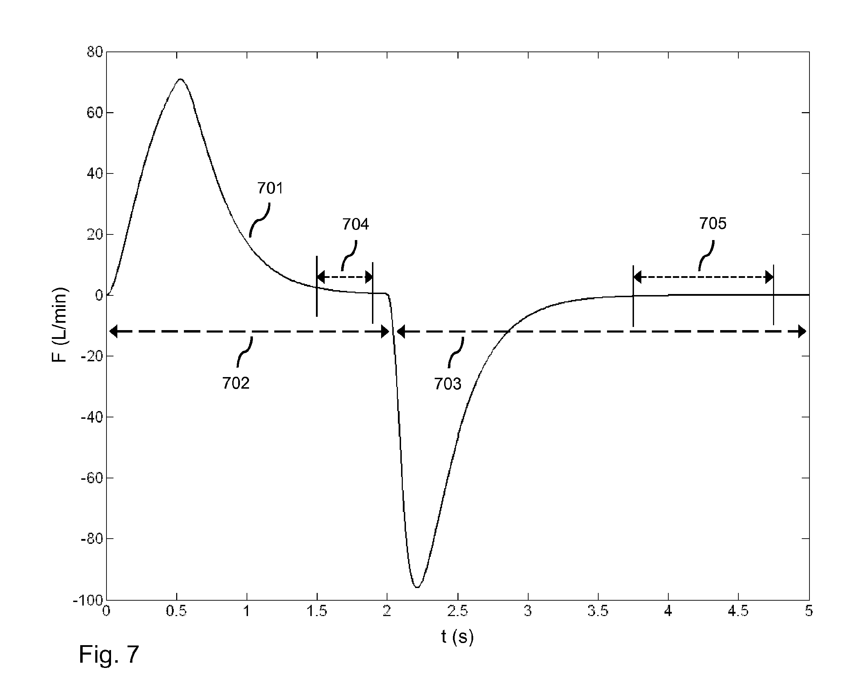

The portion of the inspiratory phase or the expiratory phase may comprise a point in a time interval from 75% to 95% of the respective phase. This is shown in Fig. 7, where the patient airway flow 701 is shown for both the inspiratory phase 702 and the expiratory phase 703. Also shown is a time interval 704 from 75% to 95% of the inspiratory phase, and a time interval 705 from 75% to 95% of the expiratory phase. Of course, other relevant time intervals may be used as well instead of the 75% to 95% interval.

The method 300 further comprises adjusting 203 the leakage parameter in dependence on the patient airway flow in an "Adjusting the leakage parameter" step to provide an adjusted leakage parameter.

This step comprises first determining 304 a summary value in dependence on the patient airway flow during a portion of a breath cycle of the patient. Here, the summary value, denoted by *S* in Fig. 3, may be an average of the patient airway flow during a portion of the expiratory phase 703 during which the patient airway flow 701 is inspiratory. The leakage parameter of the leakage model may then be adjusted 306 by decreasing 307 the leakage parameter if 305 the summary value is above an upper threshold *tᵤₚₚₑᵣ,* and increasing 308 said parameter if 305 the summary value is below a lower threshold *t_{lower}.*

The leakage parameter may also be adjusted in dependence on the inspiratory and expiratory patient airway flow during the time interval of 75% to 95% of the inspiratory and expiratory phases. Here, a value of 50 ml/min may be used as upper threshold, and a value of 10 ml/min as lower threshold. Furthermore, the leakage parameter may be increased by 0.05 if the summary value is below 10 ml/min, and decreased by 0.05 if the summary value is above 50 ml/min. Hence, the method will adjust the leakage parameter if it falls outside the desired range between 10 ml/min and 50 ml/min of patient airway flow.

Additionally, a second summary value may be determined from an average of expiratory patient airway flow during 75% to 95% of the expiratory phase. Here, if the first summary value is below 1 ml/min, and the second summary value is above - 5000 ml/min (the negative sign denoting that the airflow is expiratory, and 'above' indicating a value closer to zero), the leakage parameter may be increased by 0.10 instead of 0.05 as the values of both summary values is indicative of a leakage parameter that is particularly too low.

Similarly, a third summary value may be derived from an average of the expiratory patient airway flow during 75% to 95% of the inspiratory phase, and a fourth summary value from an average of the inspiratory patient airway flow during said phase. Here, if the third summary value is below -100 ml/min, and the fourth summary is above 10000 ml/min, the leakage parameter may be decreased by 0.05 as the values of both summary values is indicative of a leakage parameter that is particularly too high.

Of course, various alternatives to the above adjusting of the leakage parameter exist. For example, a more extensive set of thresholds may be used to more accurately adjust the leakage parameter based on said summary values, such that a large deviation of the summary value from the norm results in a large adjustment of the leakage parameter, and a small deviation from the norm results in a small adjustment of the leakage parameter.

The above adjustment may be implemented in various ways, as will be known to those skilled in the art of algorithm design. For example, the adjustment may be provided by means of a rule-based system, in which the rules reflect a certain range of the summary value and the corresponding adjustment values of the leakage parameter. Furthermore, the adjustment may be provided by a Look Up Table (LUT), in which the averaged patient airway flow is an input with which the adjustment value can be looked up in the LUT. Similarly, the adjustment may be provided by a fuzzy logic system or a single formula expressing the adjustment as a function of, amongst others, the patient airway flow.

The method 300 further comprises the step of estimating 309 the scaling factor in an "Estimating the scaling factor" step. This is done by averaging over more than one breath cycle the measured ventilation flow and the measured airway pressure, and dividing said averaged measured ventilation flow by said averaged measured airway pressure. Alternatively, this step may be omitted, and instead a fixed value for the scaling factor may be used, albeit with a decreased accuracy of the leakage model.

The method is shown in Fig. 3 to be performed more than once. In practice, this may involve that the above steps, with the possible exception of the "Estimating the scaling factor" step 309, are performed more than once during a breath cycle of the patient. The leakage flow is thus estimated in an iterative manner, which allows an adjustment of the leakage parameter during the breath cycle such that the patient airway flow falls or remains within a desired range, for example between 10 ml/min and 50 ml/min.

More typical, however, is that the method is repeated in a following breath cycle. The estimated values of the leakage model are thus used for estimating the leakage flow in an "Estimating the leakage flow" step 301 of a subsequent breath cycle. Although the characteristics of the leak may have changed between the current and the subsequent breath cycle, such change is typically slow. This holds in particular if the subsequent breath cycle is in fact the next breath cycle. In this case, the leakage model estimated for a current breath cycle typically provides a reliable estimate of the leakage flow during the next breath cycle. As such, the leakage parameter may be adjusted each breath cycle. The method thus acts as a control system that steers the estimate of the patient airway flow between a certain range.

An example of a result of the above method is shown in Fig. 5. Here, the solid line indicates a reference leakage flow 501 as provided by a simulation, the dashed line the estimated leakage flow 502 that is obtained by the above method 300 in a current cycle, and the dashed/dotted line the re-estimated leakage flow 503 by reapplying the above method 300 in a subsequent breath cycle using the adjusted leakage parameter of the current cycle.

Here, the reference leakage flow 501 is assumed to be constant between the current and the subsequent breath cycle. Clearly, the re-estimated leakage flow 503 resembles the reference leakage flow 501 more accurately than the initially estimated leakage flow 502. Fig. 4 further shows the reference patient airway flow 401, the estimated patient airway flow 402 and the re-estimated patient airway flow 403, which may be determined by subtracting from the measured ventilation flow 601 shown in Fig. 6 the reference leakage flows 501, the estimated leakage flow 502 and the re-estimated leakage flow 503 as shown in Fig. 5.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. Method (200, 300) of estimating a leakage flow (160) that occurs during ventilation of a patient (120), the method comprising:
- estimating (201, 301) the leakage flow in dependence on a measured airway pressure and a leakage parameter, the leakage parameter being indicative of a relation between the measured airway pressure and the leakage flow,
- estimating (202, 302) a patient airway flow (170) in dependence on the leakage flow and a measured ventilation flow (150),
- adjusting (203, 303) the leakage parameter in dependence on the patient airway flow to provide an adjusted leakage parameter, and
- re-estimating (211, 301) the leakage flow in dependence on the adjusted leakage parameter.

2. Method (200, 300) according to claim 1, wherein the adjusting (203, 303) the leakage parameter comprises:
- determining (204, 304) a summary value in dependence on the patient airway flow (170) during a portion of a breath cycle of the patient (120),
- comparing (205, 305) the summary value with a reference value to obtain a comparison result, and
- adjusting (206, 306) the leakage parameter in dependence on the comparison result.

3. Method (200, 300) according to claim 2, wherein the determining (204, 304) the summary value comprises averaging the patient airway flow (170) during the portion of the breath cycle of the patient (120).

4. Method (200, 300) according to any one of the above claims, wherein the estimating (201, 301) the leakage flow (160) comprises estimating the leakage flow using a leakage model in which the leakage parameter is used in an exponent of the measured airway pressure.

5. Method (200, 300) according to claim 4, wherein the leakage model comprises the equation *flowₗₑₐₖ=A·p_{aw}^{B}* in which *flowₗₐₑₖ* is the leakage flow (160), *A* a scaling factor, *p_{aw}* the measured airway pressure and *B* the leakage parameter.

6. Method (200, 300) according to any one of the above claims, wherein the estimating (202, 302) the patient airway flow (170) comprises subtracting the leakage flow (160) from the measured ventilation flow (150).

7. Method (200, 300) according to claim 2 or 3, wherein the breath cycle of the patient (120) comprises an inspiratory phase (702) and an expiratory phase (703), and wherein the determining (204, 304) the summary value comprises determining the summary value in dependence on the patient airway flow (170) during the portion of the inspiratory phase during which the patient airway flow is estimated to be expiratory, or the portion of the expiratory phase during which the patient airway flow is estimated to be inspiratory.

8. Method (200, 300) according to claim 7, wherein the portion of the inspiratory phase (702) or the expiratory phase (703) comprises a point in a time interval (704, 705) from 75% to 95% of said phase.

9. Method (300) according to claim 8, wherein the determining (304) the summary value comprises averaging the patient airway flow (170) during the portion of the expiratory phase (703) during which the patient airway flow is estimated to be inspiratory, the portion comprising the point in the time interval (705) from 75% to 95% of the expiratory phase, the comparing (305) the summary value comprising comparing the summary value with a lower reference value and with an upper reference value, and the adjusting (306) the leakage parameter comprising:
- decreasing (307) the leakage parameter if the summary value is above the upper reference value, and
- increasing (308) the leakage parameter if the summary value is below the lower reference value.

10. Method (200, 300) according to any one of the above claims, wherein the estimating (201, 301) the leakage flow (160) comprises estimating the leakage flow in dependence on the measured airway pressure and the adjusted leakage parameter provided by the re-estimating (211, 301) the leakage flow from a previous execution of said method.

11. A computer program product comprising instructions for causing a processor system to perform the method of any one of the above claims.

12. Medical device (100) for estimating a leakage flow (160) that occurs during ventilation of a patient (120), the medical device comprising:
- a first estimation unit (101) arranged for estimating the leakage flow in dependence on a measured airway pressure and a leakage parameter, the leakage parameter being indicative of a relation between the measured airway pressure and the leakage flow,
- a second estimation unit (102) arranged for estimating a patient airway flow (170) in dependence on the leakage flow and a measured ventilation flow (150),
- an adjustment unit (103) arranged for adjusting the leakage parameter in dependence on the patient airway flow to provide an adjusted leakage parameter, and
- a re-estimation unit (104) arranged for re-estimating the leakage flow in dependence on the adjusted leakage parameter.

13. Medical device (100) according to claim 12, wherein the medical device is a breathing device for non-invasive ventilation of the patient (120) by means of at least one of the group of: a breathing mask (115), a tracheastoma, a mouth piece or a non-invasive ventilation helmet.

14. Medical device (100) according to claim 12 or 13, the medical device further comprising a display (105) for providing a status indication in dependence on the leakage flow (160).
